# EUROPEAN PATENT SPECIFICATION

(11) **EP 1 317 280 B1**
(45) Date of publication and mention of the grant of the patent: **15.07.2015**
(21) Application number: 01984627.8
(22) Date of filing: 12.09.2001
(51) Int. Cl.: A61K 39/00, A61K 39/02, A61K 39/102, A61K 39/13, A61K 39/29, A61K 39/09, A61K 39/095

(54) **VACCINE AGAINST STREPTOCOCCUS PNEUMONIAE**
IMPFSTOFF GEGEN STREPTOCOCCUS PNEUMANIAE
VACCIN CONTRE STREPTOCOCCUS PNEUMONIAE

(30) Priority: 15.09.2000 GB 0022742
(43) Date of publication of application: 11.06.2003
(60) Divisional application: 09173889.8
(73) Proprietor: GlaxoSmithKline Biologicals s.a., 1330 Rixensart (BE)
(72) Inventor: HERMAND, Philippe, B-1330 Rixensart (BE); LAFERRIERE, Craig, Antony, Joseph, B-1330 Rixensart (BE); LOBET, Yves, B-1330 Rixensart (BE); POOLMAN, Jan, B-1330 Rixensart (BE)
(74) Representative: Dalton, Marcus Jonathan William
(86) International application number: PCT/EP2001/010570
(87) International publication number: WO 2002/022168

(56) References cited:
- WO-A-00/37105
- WO-A-00/76541
- WO-A-97/41151
- WO-A-98/18930
- WO-A-99/15675
- OGUNNIYI A DAVID ET AL: "Immunization of mice with combinations of pneumococcal virulence proteins elicits enhanced protection against challenge with Streptococcus pneumoniae." INFECTION AND IMMUNITY, vol. 68, no. 5, May 2000 (2000-05), pages 3028-3033, XP001064855 ISSN: 0019-9567
- DE VELASCO E.A. ET AL: "Protein-conjugated synthetic di- and trisaccharides of pneumococcal type 17F exhibit a different immunogenicity and antigenicity than tetrasaccharide", VACCINE, vol. 11, no. 14, 1 January 1993 (1993-01-01), pages 1429-1436, XP023710848, BUTTERWORTH SCIENTIFIC. GUILDFORD, GB
- WESSELS MICHAEL R. ET AL: "Structural properties of group B streptococcal type III polysaccharide conjugate vaccines that influence immunogenicity and efficacy", INFECTION AND IMMUNITY, vol. 66, no. 5, 1 May 1998 (1998-05-01), pages 2186-2192, XP009113849, AMERICAN SOCIETY FOR MICROBIOLOGY, WASHINGTON, US

## Description

### FIELD OF INVENTION

The present invention relates to a combination of 2 or more *S. pneumoniae* proteins, their manufacture and use in medicine as a vaccine. Such combinations are particularly useful for the protection of infants and elderly against streptococcal infection.

### BACKGROUND OF INVENTION

*Streptococcus pneumoniae* is a Gram-positive bacterium responsible for considerable morbidity and mortality (particularly in the young and aged), causing invasive diseases such as pneumonia, bacteremia and meningitis, and diseases associated with colonisation, such as acute Otitis media. The rate of pneumococcal pneumonia in the US for persons over 60 years of age is estimated to be 3 to 8 per 100,000. In 20% of cases this leads to bacteremia, and other manifestations such as meningitis, with a mortality rate close to 30% even with antibiotic treatment.

Pneumococcus is encapsulated with a chemically linked polysaccharide which confers serotype specificity. There are 90 known serotypes of pneumococci, and the capsule is the principle virulence determinant for pneumococci, as the capsule not only protects the inner surface of the bacteria from complement, but is itself poorly immunogenic. Polysaccharides are T-independent antigens, and can not be processed or presented on MHC molecules to interact with T-cells. They can however, stimulate the immune system through an alternate mechanism which involves cross-linking of surface receptors on B cells.

It was shown in several experiments that protection against invasive pneumococci disease is correlated most strongly with antibody specific for the capsule, and the protection is serotype specific.

*Streptococcus pneumoniae* is the most common cause of invasive bacterial disease and Otitis media in infants and young children. Likewise, the elderly mount poor responses to pneumococcal vaccines [Roghmann et al., (1987), J. Gerontol. 42:265-270], hence the increased incidence of bacterial pneumonia in this population [Verghese and Berk, (1983) Medicine (Baltimore) 62:271-285].

A 23-valent unconjugated pneumococcal vaccine has shown a wide variation in clinical efficacy, from 0% to 81% (Fedson et al. (1994) Arch Intern Med. 154: 2531-2535). The efficacy appears to be related to the risk group that is being immunised, such as the elderly, Hodgkin's disease, splenectomy, sickle cell disease and agammaglobulinemics (Fine et al. (1994) Arch Intern Med. 154:2666-2677), and also to the disease manifestation. The 23-valent vaccine does not demonstrate protection against pneumococcal pneumonia (in certain high risk groups such as the elderly) and Otitis media diseases.

Immunization of mice with combinations of pneumococcal virulence proteins is known (Ogunniyi et al., (2000), Infection and Immunity 68; 3028-3033). Vaccines comprising polyhistidine triad proteins are disclosed (WO00/37105).

Strategies, which have been designed to overcome this lack of immunogenicity in infants, include the linking of the polysaccharide to large immunogenic proteins, which provide bystander T-cell help and which induce immunological memory against the polysaccharide antigen to which it is conjugated.

However, there is still a need for improved pneumococcal vaccine compositions, particularly ones which will be more effective in the prevention or amelioration of pneumococcal disease (particularly pneumonia) in the elderly and in young children.

The present invention provides such an improved vaccine.

### SUMMARY OF THE INVENTION

In one aspect, the present invention is an immunogenic composition comprising at least 2 *S. pneumoniae* proteins as defined in claim 1.

In a related aspect, the present invention provides a vaccine for treating or ameliorating Otitis media in infants or pneumonia in the elderly. Optionally the vaccine additionally comprises an adjuvant, which is preferably an inducer of a TH1 response.

In yet another related aspect is a method for making the vaccine of the invention by selecting and isolating 2 different *S. pneumoniae* proteins and mixing both proteins with a pharmaceutically acceptable carrier as defined in the appended claims.

### DESCRIPTION OF THE INVENTION

The present invention provides an improved vaccine for the prevention or amelioration of pneumococcal infection of the elderly (e.g., pneumonia) and/or in infants (e.g., Otitis media), by relying on a pneumococcal protein based-approach. In one preferred embodiment, the vaccine is suitable for the prevention or amelioration of pneumococcal infection of the elderly. As most adults have been exposed to *Streptococcus pneumonia,* the present vaccine is intended to boost the underlying immune response in adults and the elderly to protective levels by administration of at least 2 pneumococcal proteins identified in the present invention. The pneumococcal proteins are administered in the absence of *S. pneumoniae* polysaccharides.

In the context of the present invention a patient is considered elderly if they are 55 years or over in age, typically over 60 years and more generally over 65 years. Thus in one embodiment the invention provides for a vaccine composition comprising pneumococcal proteins for the prevention of pneumonia in the elderly.

In another embodiment, the present invention provides a vaccine composition, suitable for use by infants (typically 0 to 2 years), comprising two or more pneumococcal proteins identified in the present invention.

### Pneumococcal Proteins of the disclosure

The *Streptococcus pneumoniae* proteins of the disclosure are either surface exposed, at least during part of the life cycle of the pneumococcus, or are proteins which are secreted or released by the pneumococcus. Preferably the combination of proteins of the disclosure are selected from 2 different categories such as proteins having a Type II Signal sequence motif of LXXC (where X is any amino acid, e.g., the polyhistidine triad family (PhtX)), choline binding proteins (CbpX), proteins having a Type I Signal sequence motif (e.g., Sp101), proteins having a LPXTG motif (where X is any amino acid, e.g., Sp128, Sp130), toxins (e.g., Ply), etc. Preferred examples within these categories (or motifs) are the following proteins, or immunologically functional equivalents thereof.

The immunogenic composition of the disclosure comprises at least 2 proteins selected from the group consisting of the Poly Histidine Triad family (PhtX), Choline Binding Protein family (CbpX), CbpX truncates, LytX family, LytX truncates, CbpX truncate-LytX truncate chimeric proteins (or fusions), pneumolysin (Ply), PspA, PsaA, Sp128, Sp101, Sp130, Sp125 and Sp133. However, if CbpX is PspC, then the second protein is not PspA or PsaA. Preferably, the immunogenic composition comprises 2 or more proteins selected from the group consisting of the Poly Histidine Triad family (PhtX), Choline Binding Protein family (CbpX), CbpX truncates, LytX family, LytX truncates, CbpX truncate-LytX truncate chimeric proteins (or fusions), pneumolysin (Ply), PspA, PsaA, and Sp128. More preferably, the immunogenic composition comprises 2 or more proteins selected from the group consisting of the Poly Histidine Triad family (PhtX), Choline Binding Protein family (CbpX), CbpX truncates, LytX family, LytX truncates, CbpX truncate-LytX truncate chimeric proteins (or fusions), pneumolysin (Ply), and Sp128

The Pht (Poly Histidine Triad) family comprises proteins PhtA, PhtB, PhtD, and PhtE. The family is characterised by a lipidation sequence, two domains separated by a proline-rich region and several histidine triads, possibly involved in metal or nucleoside binding or enzymatic activity, (3-5) coiled-coil regions, a conserved N-terminus and a heterogeneous C terminus. It is present in all strains of pneumococci tested. Homologous proteins have also been found in other Streptococci and Neisseria. Preferred members of the family comprise PhtA, PhtB and PhtD. More preferably, it comprises PhtA or PhtD. It is understood, however, that the terms Pht A, B, D, and E refer to proteins having sequences disclosed in the citations below as well as naturally-occurring (and man-made) variants thereof that have a sequence homology that is at least 90% identical to the referenced proteins. Preferably it is at least 95% identical and most preferably it is 97% identical.

With regards to the PhtX proteins, PhtA is disclosed in WO 98/18930, and is also referred to Sp36. As noted above, it is a protein from the polyhistidine triad family and has the type II signal motif of LXXC.

PhtD is disclosed in WO 00/37105, and is also referred to Sp036D. As noted above, it also is a protein from the polyhistidine triad family and has the type II LXXC signal motif.

PhtB is disclosed in WO 00/37105, and is also referred to Sp036B. Another member of the PhtB family is the C3-Degrading Polypeptide, as disclosed in WO 00/17370. This protein also is from the polyhistidine triad family and has the type II LXXC signal motif. A preferred immunologically functional equivalent is the protein Sp42 disclosed in WO 98/18930. A PhtB truncate (approximately 79kD) is disclosed in WO99/15675 which is also considered a member of the PhtX family.

PhtE is disclosed in WO00/30299 and is referred to as BVH-3.

Concerning the Choline Binding Protein family (CbpX), members of that family were originally identified as pneumococcal proteins that could be purified by choline-affininty chromatography. All of the choline-binding proteins are non-covalently bound to phosphorylcholine moieties of cell wall teichoic acid and membrane-associated lipoteichoic acid. Structurally, they have several regions in common over the entire family, although the exact nature of the proteins (amino acid sequence, length, etc.) can vary. In general, choline binding proteins comprise an N terminal region (N), conserved repeat regions (R1 and/or R2), a proline rich region (P) and a conserved choline binding region (C), made up of multiple repeats, that comprises approximately one half of the protein. As used in this application, the term "Choline Binding Protein family (CbpX)" is selected from the group consisting of Choline Binding Proteins as identified in WO97/41151, PbcA, SpsA, PspC, CbpA, CbpD, and CbpG. CbpA is disclosed in WO97/41151. CbpD and CbpG are disclosed in WO00/29434. PspC is disclosed in WO97/09994. PbcA is disclosed in WO98/21337.SpsA is a Choline binding protein disclosed in WO 98/39450. Preferably the Choline Binding Proteins are selected from the group consisting of CbpA, PbcA, SpsA and PspC.

Another preferred embodiment is CbpX truncates wherein "CbpX" is defined above and "truncates" refers to CbpX proteins lacking 50% or more of the Choline binding region (C). Preferably such proteins lack the entire choline binding region. More preferably, the such protein truncates lack (i) the choline binding region and (ii) a portion of the N-terminal half of the protein as well, yet retain at least one repeat region (R1 or R2). More preferably still, the truncate has 2 repeat regions (R1 and R2). Examples of such preferred embodiments are NR1xR2 and R1xR2 as illustrated in WO99/51266 or WO99/51188, however, other choline binding proteins lacking a similar choline binding region are also contemplated within the scope of this disclosure.

The LytX family is membrane associated proteins associated with cell lysis. The N-terminal domain comprises choline binding domain(s), however the LytX family does not have all the features found in the CbpA family noted above and thus for the present invention, the LytX family is considered distinct from the CbpX family. In contrast with the CbpX family, the C-terminal domain contains the catalytic domain of the LytX protein family. The family comprises LytA, B and C. With regards to the LytX family, LytA is disclosed in Ronda et al., Eur J Biochem, 164:621-624 (1987). LytB is disclosed in WO 98/18930, and is also referred to as Sp46. LytC is also disclosed in WO 98/18930, and is also referred to as Sp91. A preferred member of that family is LytC.

Another preferred embodiment are LytX truncates wherein "LytX" is defined above and "truncates" refers to LytX proteins lacking 50% or more of the Choline binding region. Preferably such proteins lack the entire choline binding region. An example of such truncates can be found in the Examples section of this disclosure.

Yet another preferred embodiment of this disclosure are CbpX truncate-LytX truncate chimeric proteins (or fusions). Preferably this comprises NR1xR2 (or R1xR2) of CbpX and the C-terminal portion (Cterm, i.e., lacking the choline binding domains) of LytX (e.g., LytCCterm or Sp91Cterm). More preferably CbpX is selected from the group consisting of CbpA, PbcA, SpsA and PspC. More preferably still, it is CbpA. Preferably, LytX is LytC (also referred to as Sp91).

Another embodiment of the present disclosure is a PspA or PsaA truncates lacking the choline binding domain (C) and expressed as a fusion protein with LytX. Preferably, LytX is LytC.

Pneumolysin is a multifunctional toxin with a distinct cytolytic (hemolytic) and complement activation activities (Rubins et al., Am. Respi. Cit Care Med, 153:1339-1346 (1996)). The toxin is not secreted by pneumococci, but it is released upon lysis of pneumococci under the influence of autolysin. Its effects include e.g., the stimulation of the production of inflammatory cytokines by human monocytes, the inhibition of the beating of cilia on human respiratory epithelial, and the decrease of bactericidal activity and migration of neutrophils. The most obvious effect of pneumolysin is in the lysis of red blood cells, which involves binding to cholesterol. Because it is a toxin, it needs to be detoxified (i.e., non-toxic to a human when provided at a dosage suitable for protection) before it can be administered *in vivo.* Expression and cloning of wild-type or native pneumolysin is known in the art. See, for example, Walker et al. (Infect Immun, 55:1184-1189 (1987)), Mitchell et al. (Biochim Biophys Acta, 1007:67-72 (1989) and Mitchell et al (NAR, 18:4010 (1990)). Detoxification of ply can be conducted by chemical means, e.g., subject to formalin or glutarahdehye treatment or a combination of both. Such methods are well known in the art for various toxins. Alternatively, ply can be genetically detoxified. Thus, the invention encompasses derivatives of pneumococcal proteins which may be, for example, mutated proteins. The term "mutated" is used herein to mean a molecule which has undergone deletion, addition or substitution of one or more amino acids using well known techniques for site directed mutagenesis or any other conventional method. For example, as described above, a mutant ply protein may be altered so that it is biologically inactive whilst still maintaining its immunogenic epitopes, see, for example, WO90/06951, Berry et al. (Infect Immun, 67:981-985 (1999)) and WO99/03884.

As used herein, it is understood that the term "Ply" refers to mutated or detoxified pneumolysin suitable for medical use (i.e., non toxic).

With regards to PsaA and PspA, both are know in the art. For example, PsaA and transmembrane deletion variants thereof have been described by Berry & Paton, Infect Immun 1996 Dec;64(12):5255-62. PspA and transmembrane deletion variants thereof have been disclosed in, for example, US 5804193, WO 92/14488, and WO 99/53940.

Sp128 and Sp130 are disclosed in WO00/76540.

Sp125 is an example of a pneumococcal surface protein with the Cell Wall Anchored motif of LPXTG (where X is any amino acid). Any protein within this class of pneumococcal surface protein with this motif has been found to be useful within the context of this disclosure and is therefore considered a further protein of the invention. Sp125 itself is disclosed in WO 98/18930, and is also known as ZmpB - a zinc metalloproteinase.

Sp101 is disclosed in WO 98/06734 (where it has the reference # y85993). It is characterised by a Type I signal sequence.

Sp133 is disclosed in WO 98/06734 (where it has the reference # y85992). It is also characterised by a Type I signal sequence.

The proteins of the disclosure may also be beneficially combined. Preferred combinations include, but are not limited to, PhtD + NR1xR2, PhtD + NR1xR2-Sp91Cterm chimeric or fusion proteins, PhtD + Ply, PhtD + Sp128, PhtD + PsaA, PhtD + PspA, PhtA + NR1xR2, PhtA + NR1xR2-Sp91Cterm chimeric or fusion proteins, PhtA + Ply, PhtA + Sp128, PhtA + PsaA, PhtA + PspA, NR1xR2 + LytC, NR1xR2 + PspA, NR1xR2 + PsaA, NR1xR2 + Sp128, R1xR2 + LytC, R1xR2 + PspA, R1xR2 + PsaA, R1xR2 + Sp128, R1xR2 + PhtD, R1xR2 + PhtA. Preferably, NR1xR2 (or R1xR2) is from CbpA or PspC. More preferably it is from CbpA.

A particularly preferred combination of pneumococcal proteins comprises Ply (or a truncate or immunologically functional equivalent thereof) + PhtD (or a truncate or immunologically functional equivalent thereof) + NR1xR2 (or R1xR2). Preferably, NR1xR2 (or R1xR2) is from CbpA or PspC. More preferably it is from CbpA.

The present disclosure also encompasses "immunologically functional equivalent(s) to the proteins of the disclosure "Immunologically functional equivalent(s)" is defined as a peptide or protein comprising at least one protective epitope from the proteins of the disclosure. Such epitopes are characteristically surface-exposed, highly conserved, and can elicit a bactericidal antibody response in a host or prevent toxic effects. Preferably, the functional equivalent has at least 15 and preferably 30 or more contiguous amino acids from the protein of the disclosure can be used with the proviso that they are capable of raising substantially the same immune response as the native protein. The position of potential B-cell epitopes in a protein sequence may be readily determined by identifying peptides that are both surface-exposed and antigenic using a combination of two methods: 2D-structure prediction and antigenic index prediction. The 2D-structure prediction can be made using the PSIPRED program (from David Jones, Brunel Bioinformatics Group, Dept. Biological Sciences, Brunel University, Uxbridge UB8 3PH, UK). The antigenic index can be calculated on the basis of the method described by Jameson and Wolf (CABIOS 4:181-186 [1988]).

The present disclosure has advantages over *S. pneumoniae* polysaccharide vaccines in that multiple *S. pneumoniae* protein (immunogenic) compositions may include greater cross-protection across the numerous serotypes, can further inhibit adherence and colony formulation, and can potentially can raise antibodies that can neutralise the toxic/enzymatic functions of a pathogen. Furthermore, additional surface antigens provide a means to further stimulate opsonophagocytosis.

The present disclosure also contemplates combination vaccines which provide protection against a range of different pathogens. Many Paediatric vaccines are now given as a combination vaccine so as to reduce the number of injections a child has to receive. Thus for Paediatric vaccines other antigens from other pathogens may be formulated with the vaccines of the disclosure. For example the vaccines of the invention can be formulated with (or administered separately but at the same time) the well known 'trivalent' combination vaccine comprising Diphtheria toxoid (DT), tetanus toxoid (TT), and pertussis components [typically detoxified Pertussis toxoid (PT) and filamentous haemagglutinin (FHA) with optional pertactin (PRN) and/or agglutinin 1+2], for example the marketed vaccine INFANRIX-DTPa™ (SmithKlineBeecham Biologicals) which contains DT, TT, PT, FHA and PRN antigens, or with a whole cell pertussis component for example as marketed by SmithKlineBeecham Biologicals s.a., as Tritanrix™. The combined vaccine may also comprise other antigen, such as Hepatitis B surface antigen (HBsAg), Polio virus antigens (for instance inactivated trivalent polio virus - IPV), *Moraxella catarrhalis* outer membrane proteins, non-typeable *Haemophilus influenzae* proteins, *N.meningitidis* B outer membrane proteins.

Examples of preferred *Moraxella catarrhalis* protein antigens which can be included in a combination vaccine (especially for the prevention of otitis media) are: OMP106 [WO 97/41731 (Antex) & WO 96/34960 (PMC)]; OMP21; LbpA &/or LbpB [WO 98/55606 (PMC)]; TbpA &/or TbpB [WO 97/13785 & WO 97/32980 (PMC)]; CopB [Helminen ME, et al. (1993) Infect. Immun. 61:2003-2010]; UspA1 and/or UspA2 [WO 93/03761 (University of Texas)]; OmpCD; HasR (PCT/EP99/03824); PilQ (PCT/EP99/03823); OMP85 (PCT/EP00/01468); lipo06 (GB 9917977.2); lipo10 (GB 9918208.1); lipo11 (GB 9918302.2); lipo18 (GB 9918038.2); P6 (PCT/EP99/03038); D15 (PCT/EP99/03822); Omp1A1 (PCT/EP99/06781); Hly3 (PCT/EP99/03257); and OmpE. Examples of non-typeable *Haemophilus influenzae* antigens which can be included in a combination vaccine (especially for the prevention of otitis media) include: Fimbrin protein [(US 5766608 - Ohio State Research Foundation)] and fusions comprising peptides therefrom [eg LB1(f) peptide fusions; US 5843464 (OSU) or WO 99/64067]; OMP26 [WO 97/01638 (Cortecs)]; P6 [EP 281673 (State University of New York)]; TbpA and/or TbpB; Hia; Hsf; Hin47; Hif; Hmw1; Hmw2; Hmw3; Hmw4; Hap; D15 (WO 94/12641); protein D (EP 594610); P2; and P5 (WO 94/26304).

In another embodiment, various antigens recited above can be included in the immunogenic composition as antigens present on the surface of outer membrane vesicles (blebs) made from the bacteria from which it is derived.

Other combinations contemplated are the *S. pneumoniae* proteins in combination with viral antigens, for example, from influenza (attenuated, split, or subunit [e.g., surface glycoproteins neuraminidase (NA) and haemagglutinin (HA). See, e.g., Chaloupka I. et al, Eur. Journal Clin. Microbiol. Infect. Dis. 1996, 15:121-127], RSV (e.g., F and G antigens or F/G fusions, see, eg, Schmidt A. C. et al, J Virol, May 2001, p4594 - 4603), PIV3 (e.g., HN and F proteins, see Schmidt et al. supra), Varicella (e.g., attenuated, glycoproteins I-V, etc.), and any (or all) component(s) of MMR (measles, mumps, rubella).

### Polysaccharide Antigens of the disclosure

The present application also contemplates combination vaccines with 2 or more *S. pneumoniae* proteins combined with polysaccharides other than from *S*. *pneumonaie.* Such polysaccharides can be isolated from, for example, H. influenzae, H. influenzae type B (Hib), N. meningitidis groups A, C, W, Y, Streptococci other than *S. pneumoniae* (e.g., Group B Streptococcus, *S. pyogenes,* etc.), Staphylococcus (e.g., *S. aureus, S. epidermidis), E. coli,* Enterococcus (e.g., *E. faecalis* and *E*. *faecium*) ,etc. Preferably the polysaccharides are from H. influenzae type B (Hib), and/or N. meningitidis groups A, C, W135, and/or Y.

As mentioned above, a problem associated with the polysaccharide approach to vaccination, is the fact that polysaccharides *per se* are poor immunogens. To overcome this, polysaccharides may be conjugated to protein carriers, which provide bystander T-cell help. It is preferred, therefore, that the polysaccharides utilised are linked to such a protein carrier. Examples of such carriers which are currently commonly used for the production of polysaccharide immunogens include the Diphtheria and Tetanus toxoids (DT, DT CRM197, other DT mutants, e.g. position Glu-148, etc. [see, e.g., US 4,709,017, WO93/25210, WO95/33481, etc.] and TT (and TT fragment C) respectively), Keyhole Limpet Haemocyanin (KLH), OMPC from *N. meningitidis,* and the purified protein derivative of Tuberculin (PPD).

Another carrier for the polysaccharide based immunogenic compositions (or vaccines) is protein D from *Haemophilus influenzae* (EP 594610-B), or fragments thereof. Fragments suitable for use include fragments encompassing T- helper epitopes. In particular a protein D fragment will preferably contain the N-terminal 1/3 of the protein.

The polysaccharide may be linked to the carrier protein by any known method (for example, by Likhite, U.S. Patent 4,372,945 and by Armor et al., U.S. Patent 4,474,757). Preferably, CDAP conjugation is carried out (WO 95/08348). To enhance immunogenicity, the polysaccharides may be sized (depolymerized), adjuvanted, lyophilised, or be conjugated to different carrier proteins.

### TH1 Adjuvants of the Disclosure

The vaccines of the present disclosure are preferably adjuvanted. Suitable adjuvants include an aluminium salt such as aluminium hydroxide gel (alum) or aluminium phosphate, but may also be a salt of calcium, magnesium, iron or zinc, or may be an insoluble suspension of acylated tyrosine, or acylated sugars, cationically or anionically derivatised polysaccharides, or polyphosphazenes.

It is preferred that the adjuvant be selected to be a preferential inducer of a TH1 type of response. Such high levels of Th1-type cytokines tend to favour the induction of cell mediated immune responses to a given antigen, whilst high levels of Th2-type cytokines tend to favour the induction of humoral immune responses to the antigen.

It is important to remember that the distinction of Th1 and Th2-type immune response is not absolute. In reality an individual will support an immune response which is described as being predominantly Th1 or predominantly Th2. However, it is often convenient to consider the families of cytokines in terms of that described in murine CD4 +ve T cell clones by Mosmann and Coffman (Mosmann, T.R. and Coffman, R.L. (1989) TH1 and TH2 cells: different patterns of lymphokine secretion lead to different functional properties. Annual Review of Immunology, 7, p145-173). Traditionally, Th1-type responses are associated with the production of the INF-γ and IL-2 cytokines by T-lymphocytes. Other cytokines often directly associated with the induction of Th1-type immune responses are not produced by T-cells, such as IL-12. In contrast, Th2-type responses are associated with the secretion of Il-4, IL-5, IL-6, IL-10. Suitable adjuvant systems which promote a predominantly Th1 response include: Monophosphoryl lipid A or a derivative thereof, particularly 3-de-O-acylated monophosphoryl lipid A (3D-MPL) (for its preparation see GB 2220211 A); and a combination of monophosphoryl lipid A, preferably 3-de-O-acylated monophosphoryl lipid A, together with either an aluminium salt (for instance aluminium phosphate or aluminium hydroxide) or an oil-in-water emulsion. In such combinations, antigen and 3D-MPL are contained in the same particulate structures, allowing for more efficient delivery of antigenic and immunostimulatory signals. Studies have shown that 3D-MPL is able to further enhance the immunogenicity of an alum-adsorbed antigen [Thoelen et al. Vaccine (1998) 16:708-14; EP 689454-B1].

An enhanced system involves the combination of a monophosphoryl lipid A and a saponin derivative, particularly the combination of QS21 and 3D-MPL as disclosed in WO 94/00153, or a less reactogenic composition where the QS21 is quenched with cholesterol as disclosed in WO 96/33739.

A particularly potent adjuvant formulation involving QS21, 3D-MPL and tocopherol in an oil in water emulsion is described in WO 95/17210, and is a preferred formulation.

Preferably the vaccine additionally comprises a saponin, more preferably QS21. The formulation may also comprise an oil in water emulsion and tocopherol (WO 95/17210).

The present disclosure also provides a method for producing a vaccine formulation comprising mixing a protein of the present invention together with a pharmaceutically acceptable excipient, such as 3D-MPL.

Unmethylated CpG containing oligonucleotides (WO 96/02555) are also preferential inducers of a TH1 response and are suitable for use in the present invention.

In a further aspect of the present invention there is provided a vaccine as herein described for use in medicine. In one embodiment there is a method of preventing or ameliorating pneumonia in an elderly human comprising administering a safe and effective amount of a vaccine of the invention, and optionally a Th1 adjuvant, to said elderly patient.

In a further embodiment there is provided a method of preventing or ameliorating otitis media in Infants (up to 24 months) or toddlers (typically 24 months to 5 years), comprising administering a safe and effective amount of a vaccine comprising a *Streptococcus pneumoniae* proteins of the invention and optionally a Th1 adjuvant, to said Infant or toddler.

### Vaccine Preparations of the disclosure

The vaccine preparations of the present invention may be used to protect or treat a mammal (preferably a human patient) susceptible to infection, by means of administering said vaccine via systemic or mucosal route. These administrations may include injection *via* the intramuscular, intraperitoneal, intradermal or subcutaneous routes; or *via* mucosal administration to the oral/alimentary, respiratory, genitourinary tracts. Intranasal administration of vaccines for the treatment of pneumonia or otitis media is preferred (as nasopharyngeal carriage of pneumococci can be more effectively prevented, thus attenuating infection at its earliest stage). Although the vaccine of the invention may be administered as a single dose, components thereof may also be co-administered together at the same time or at different times (for instance if polysaccharides are present in a vaccine these could be administered separately at the same time or 1-2 weeks after the administration of the bacterial protein combination for optimal coordination of the immune responses with respect to each other). In addition to a single route of administration, 2 different routes of administration may be used. For example, viral antigens may be administered ID (intradermal), whilst bacterial proteins may be administered IM (intramuscular) or IN (intranasal). If polysaccharides are present, they may be administered IM (or ID) and bacterial proteins may be administered IN (or ID). In addition, the vaccines of the invention may be administered IM for priming doses and IN for booster doses.

The amount of conjugate antigen in each vaccine dose is selected as an amount which induces an immunoprotective response without significant, adverse side effects in typical vaccines. Such amount will vary depending upon which specific immunogen is employed and how it is presented. The content of protein antigens in the vaccine will typically be in the range 1-100µg, preferably 5-50µg, most typically in the range 5 - 25µg. If polysaccharides are included, generally it is expected that each dose will comprise 0.1-100 µg of polysaccharide, preferably 0.1-50 µg, more preferably 0.1-10 µg, of which 1 to 5 µg is the most preferable range.

Optimal amounts of components for a particular vaccine can be ascertained by standard studies involving observation of appropriate immune responses in subjects. Following an initial vaccination, subjects may receive one or several booster immunisations adequately spaced. Typically a vaccine will comprise antigen (proteins), an adjuvant, and excipients or a pharmaceutically acceptable carrier.

Vaccine preparation is generally described in Vaccine Design ("The subunit and adjuvant approach" (eds Powell M.F. & Newman M.J.) (1995) Plenum Press New York). Encapsulation within liposomes is described by Fullerton, US Patent 4,235,877.

Although the vaccines of the present invention may be administered by any route, administration of the described vaccines into the skin (ID) forms one embodiment of the present disclosure Human skin comprises an outer "horny" cuticle, called the stratum corneum, which overlays the epidermis. Underneath this epidermis is a layer called the dermis, which in turn overlays the subcutaneous tissue. Researchers have shown that injection of a vaccine into the skin, and in particular the dermis, stimulates an immune response, which may also be associated with a number of additional advantages. Intradermal vaccination with the vaccines described herein forms a preferred feature of the present disclosure.

The conventional technique of intradermal injection, the "mantoux procedure", comprises steps of cleaning the skin, and then stretching with one hand, and with the bevel of a narrow gauge needle (26-31 gauge) facing upwards the needle is inserted at an angle of between 10-15°. Once the bevel of the needle is inserted, the barrel of the needle is lowered and further advanced whilst providing a slight pressure to elevate it under the skin. The liquid is then injected very slowly thereby forming a bleb or bump on the skin surface, followed by slow withdrawal of the needle.

More recently, devices that are specifically designed to administer liquid agents into or across the skin have been described, for example the devices described in WO 99/34850 and EP 1092444, also the jet injection devices described for example in WO 01/13977; US 5,480,381, US 5,599,302, US 5,334,144, US 5,993,412, US 5,649,912, US 5,569,189, US 5,704,911, US 5,383,851, US 5,893,397, US 5,466,220, US 5,339,163, US 5,312,335, US 5,503,627, US 5,064,413, US 5,520, 639, US 4,596,556, US 4,790,824, US 4,941,880, US 4,940,460, WO 97/37705 and WO 97/13537. Alternative methods of intradermal administration of the vaccine preparations may include conventional syringes and needles, or devices designed for ballistic delivery of solid vaccines (WO 99/27961), or transdermal patches (WO 97/48440; WO 98/28037); or applied to the surface of the skin (transdermal or transcutaneous delivery WO 98/20734 ; WO 98/28037).

When the vaccines of the present invention are to be administered to the skin, or more specifically into the dermis, the vaccine is in a low liquid volume, particularly a volume of between about 0.05 ml and 0.2 ml.

The content of antigens in the skin or intradermal vaccines may be similar to conventional doses as found in intramuscular vaccines. Accordingly, the protein antigens present in the intradermal vaccines may in the range 1-100µg, preferably 5-50µg. Likewise, if present, the amount of polysaccharide conjugate antigen in each vaccine dose is generally expected to comprise 0.1-100 µg of polysaccharide, preferably 0.1-50 µg, preferably 0.1-10 µg, and may be between 1 and 5 µg. However, it is a feature of skin or intradermal vaccines that the formulations may be "low dose". Accordingly the protein antigens in "low dose" vaccines are preferably present in as little as 0.1 to 10µg, preferably 0.1 to 5 µg per dose; and if present the polysaccharide conjugate antigens may be present in the range of 0.01-1µg, and preferably between 0.01 to 0.5 µg of polysaccharide per dose.

As used herein, the term "intradermal delivery" means delivery of the vaccine to the region of the dermis in the skin. However, the vaccine will not necessarily be located exclusively in the dermis. The dermis is the layer in the skin located between about 1.0 and about 2.0 mm from the surface in human skin, but there is a certain amount of variation between individuals and in different parts of the body. In general, it can be expected to reach the dermis by going 1.5 mm below the surface of the skin. The dermis is located between the stratum corneum and the epidermis at the surface and the subcutaneous layer below. Depending on the mode of delivery, the vaccine may ultimately be located solely or primarily within the dermis, or it may ultimately be distributed within the epidermis and the dermis.

In another aspect the present disclosure may contain DNA encoding one or more S. pneumoniae proteins, such that the protein is generated in situ. The DNA may be present within any of a variety of delivery systems known to those of ordinary skill in the art, including nucleic acid expression systems, bacteria and viral expression systems. Numerous gene delivery techniques are well known in the art, such as those described by Rolland, (Crit. Rev. Therap. Drug Carrier Systems 15:143-198, 1998) and references cited therein. Appropriate nucleic acid expression systems contain the necessary DNA sequences for expression in the patient (such as a suitable promoter and terminating signal). When the expression system is a recombinant live microorganism, such as a virus or bacterium, the gene of interest can be inserted into the genome of a live recombinant virus or bacterium. Inoculation and in vivo infection with this live vector will lead to in vivo expression of the antigen and induction of immune responses. Viruses and bacteria used for this purpose are for instance: poxviruses (e.g; vaccinia, fowlpox, canarypox), alphaviruses (Sindbis virus, Semliki Forest Virus, Venezuelian Equine Encephalitis Virus), adenoviruses, adeno-associated virus, picornaviruses (poliovirus, rhinovirus), herpesviruses (varicella zoster virus, etc), Listeria, Salmonella, Shigella, Neisseria, BCG. These viruses and bacteria can be virulent, or attenuated in various ways in order to obtain live vaccines. Such live vaccines also form part of the invention.

In a further aspect of the present invention there is provided a method of manufacture of a vaccine formulation as herein described, wherein the method comprises mixing a combination of proteins according to the invention.

Preferably the antigenic compositions (and vaccines) that contain polysaccharides hereinbefore described are lyophilised up until they are about to be used, at which point they are extemporaneously reconstituted with diluent. More preferably they are lyophilised in the presence of 3D-MPL, and are extemporaneously reconstituted with saline solution.

The lyophilisation of vaccines is well known in the art. Typically the liquid vaccine is freeze dried in the presence of an anti-caking agent for instance sugars such as sucrose or lactose (present at an initial concentration of 10-200 mg/mL). Lyophilisation typically occurs over a series of steps, for instance a cycle starting at-69 °C, gradually adjusting to -24 °C over 3 hours, then retaining this temperature for 18 hours, then gradually adjusting to -16 °C over 1 hour, then retaining this temperature for 6 hours, then gradually adjusting to +34 °C over 3 hours, and finally retaining this temperature over 9 hours.

The immunogenic compositions and vaccines can be evaluated in various animal models or with human sera. As an illustration, the following animal models can be used to evaluate pneumococcal infection. C3H/HeJ Mice (6 to 8 week old) can be immunised s.c. with 15 µg protein adjuvanted with 50 µl CFA, followed 3-4 weeks later by boosting with 15 µg protein with IFA. For demonstrating passive and active protection from systemic infection, mice can be administered intraperitoneally with immune sera or proteins prior to challenge by intraperitoneal injection with 15 to 90 LD50 pneumococci on week 8-10. Additionally, proteins can be tested in a mouse nasopharynx colonization model by (Wu et al Microbial Pathogenesis 1997; 23:127-137).

In addition to mice, infant rats are susceptible to colonisation and infection by *S. pneumoniae.* In passive protective studies, administration of mouse immune sera (100 ul i.p. or 10 ul i.n.) can be done prior to challenge with intranasal administration of *S.pneumonia* (10 ul) in 2-5 day old infant rat pups. Colonisation can be determined by plating nasal washes (20-40 ul instilled, 10 ul withdrawn).

Favourable interactions between the protein components of the combination vaccine may be demonstrated by administering a dose of each protein in the vaccine which would be sub-protective in a monovalent vaccine. Increased protective efficacy of the combination vaccine compared to monovalent vaccines can be attributed to a favourable interaction between the components.

The invention is illustrated in the accompanying examples. The examples are carried out using standard techniques, which are well known and routine to those of skill in the art, except where otherwise described in detail. The examples are meant to illustrate, but not limit the invention.

### EXAMPLES

### Example 1. Construction and Expression of Antigens

### NR1xR2

CbpA is a 75kDa surface-exposed protein consisting of several domains. The N-terminal domain comprises 2 highly conserved repeats (R1 and R2) and the C-terminal domain comprises 10 tandem, direct repetitive sequences of 20 amino acids. A CbpA truncate was prepared to produce NR1XR2, i.e., without the choline binding domain.

The NR1XR2 gene was amplified, via PCR, from DNA obtained from a serotype 4 strain of S. pneumoniae (see, e.g., WO97/41157, or WO99/51266). PCR was performed with the Expand High Fidelity PCR System, or Hi-Fi (Roche). It's composed of a mix containing Taq polymerase and a proofreading polymerase. Due to the inherent 3'-5' exonuclease proofreading activity, the use of Hi-Fi results in a 3 fold increased fidelity of DNA synthesis compared to Taq polymerase.

PCR fragments were cloned in pGEM-T vector from pGEM-T Vector Systems (Promega). This step is needed to facilitate restriction enzyme digestion of PCR fragment for future ligation. pGEM-T vector is provided linear and contains 3'-T overhangs. These overhangs facilitate insertion of PCR products generated by thermostable polymerases that add a single deoxyadenosine, in a template-independent fashion, to the 3' ends of the amplified fragment

Fragments and vectors were purified after enzymatic digestions (*NdeI* and *XbaI* digestions) according to the article of Benore-Parsons et al. (Nucleic Acids research, 23, 4926-4927, 1995). Agarose slice was completed lyophilized during 3-4 hours. A 1:1 ethanol-TE solution was added to the lyophilised gel. The sample was gently mixed for 1h, the agarose was compressed and completely removed by centrifugation. DNA was recovered from the eluant by ethanol precipitation.

The DNA encoding NR1xR2 was cloned into a vector containing long promoter L from phage λ. The protein of interest could be induced by heat when present in AR 58 *E. coli* strain, or by nalidixic acid in AR 120 *E. coli* strain.

A preculture of bacteria was made overnight at 30°C. This preculture was diluted about 40 times in a total volume of 20 ml and put at 30°C until an O.D. of 0.4-0.6. Then, heat induction was made at 42°C. Samples were taken at different time points. One ml of culture was centrifuged 5 minutes at 7000 rpm. Culture supernatant was conserved at -20°C and pellet (total extract) was resuspended in 500 µl of sample buffer (western blot or SDS-PAGE analysis), or in 500 µl of lysis buffer and incubated 30 minutes at 37°C (ELISA). The composition of lysis buffer is: SDS 0.1 %, Deoxycholate 0.1 %, Na citrate: 0.015 M.

Samples were run on a SDS-PAGE, loaded on 4-20 % gel (Novex, Invitrogen). Migration was done at 200 V. Coomassie blue staining was performed. Samples were loaded on 4-20 % gel (Novex, Invitrogen) for Western Blotting. Migration was done at 200 V. Gel was transferred on nitrocellulose and spots were revealed with rabbit α-NR1XR2 polyclonal antibodies (first antibody) and a α-rabbit antibody coupled to alkaline phosphatase (second antibody).

A band of approximately 55kDa was observed by SDS-PAGE analysis. A clone, 28B2, was chosen on basis of the SDS-PAGE analysis and transferred to fermentation. This clone was sequenced and its sequence was confirmed (amino acids 39 (i.e., after signal sequence) to 446 = 406 amino acids).

The solubility of NR1XR2 was also studied, after lysis of overnight-induced bacteria followed by centrifugation. A SDS-PAGE analysis and an ELISA test were performed. NR1XR2 appeared to be mainly (> 95%) recovered in the soluble fraction.

### R1xR2, PhtD, Sp91, (N)R1xR2-Sp91[C-terminal domain], and Ply

These genes were also cloned, sequenced and expressed in a similar manner to NR1xR2. R1xR2 contains amino acids 177 to 443 of CbpA (of *S. pneumoniae* serotype 4N), PhtD contains amino acids 21 (i.e., after signal sequence) to the end (amino acid 839 of *S. pneumoniae* serotype 4N), Sp91 starts at amino acid 20 (VAA) till the end. For the fusion proteins, R1xR2-Sp91Cterm contains amino acids 177-446 of CbpA and amino acids 271 until the translation stop; NR1xR2-Sp91Cterm contains amino acids 39-446 of CbpA and amino acids 271 until the translation stop. For both fusion proteins, 2 additional amino acids (GS) are found between the (N)R1xR2 and Sp91Cterm sequences. For all constructs, an ATG has been introduced 5' of the gene start to allow transcription and translation, which means that there is an additional N-terminus methionine in front of each sequence mentioned above.

### Example 2. Serology

Using sera from clinical studies, ELISAs were measured for the antibody response that naturally developed to *S. pneumoniae* proteins.

### 2.1 EXPERIMENTAL PROCEDURE

### • Serum samples

□ Paired sera of infants collected when they were 2 to 4 months old and 6 to 12 months old, respectively (N = 20, studies DTPa HBV).
□ Sera of -20-year-old adults (N = 50).
□ Sera of ≥ 65-year-old adults (N =140).

### • ELISA procedures

Immuno-plates were coated overnight at 4°C with 1 µg/ml of each protein. Serial twofold dilutions of sera (starting at a 1/10^{th} dilution) were then incubated for 1 hour at room temperature (RT) under shaking. Immuno-detection was done using a peroxydase-coupled anti-human IgG monoclonal antibody (Strateck, HP6043) diluted 4000-fold and incubated for 30 minutes at RT under shaking. After revelation, midpoint titers were calculated by SoftMaxPro. Sera with titers ≥ 10 were considered as positive. For the geometric mean calculation, a titer of 5 (half of the cut-off) was arbitrarily attributed to negative sera.

IgG concentrations expressed as µg/ml were established by comparing sample optical densities (OD) to the OD curve of chromopure IgG (Jackson) entrapped on the plate by polyclonal anti-human IgG goat antibodies and revealed by the same peroxydase-labeled antibody as above.

### 2.2 RESULTS

### 2.2.1 Strep protein serology in infants

The highest antibody titers and seropositivity rates measured in sera of 2 to 4-month-old infants were obtained with PhtD, PsaA, Sp128, NR1xR2 and in a lesser extent, with Sp91 and Ply. No or low responses to SplOI and Sp130 were detected. Sp46 and PhtA were not tested (material availability issue).

The antibody responses generally decreased in sera of the same subjects collected when these were 6 to 12 months old, suggesting the high titers were mainly due to passively transferred maternal antibodies.

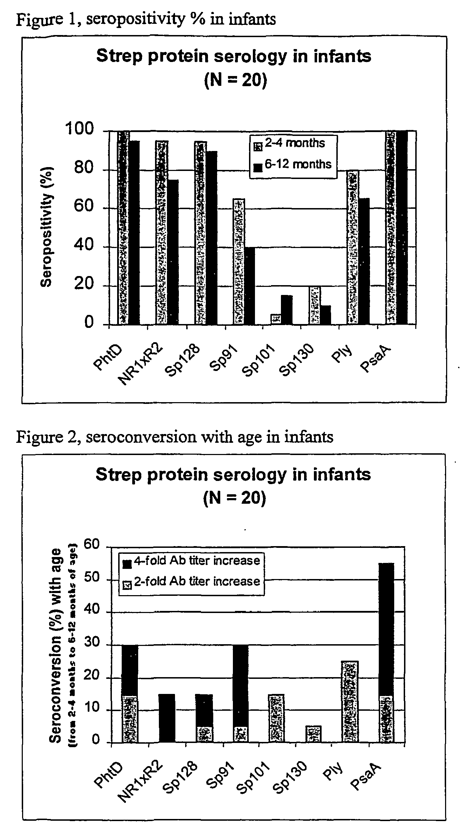

However, in certain infants, the immune response to some proteins increased with age, probably as a consequence of natural exposure to pneumococci. The antigen mainly concerned by this seroconversion was clearly PsaA. Depending on the subject augmentation of antibody levels to PhtD, NR1xR2, Sp128, Sp91 and Ply was also shown. Only marginal variation in the humoral response to Sp101 and Sp130 was observed. (See figures 1 and 2)

### 2.2.2 Strep protein serology in young adults

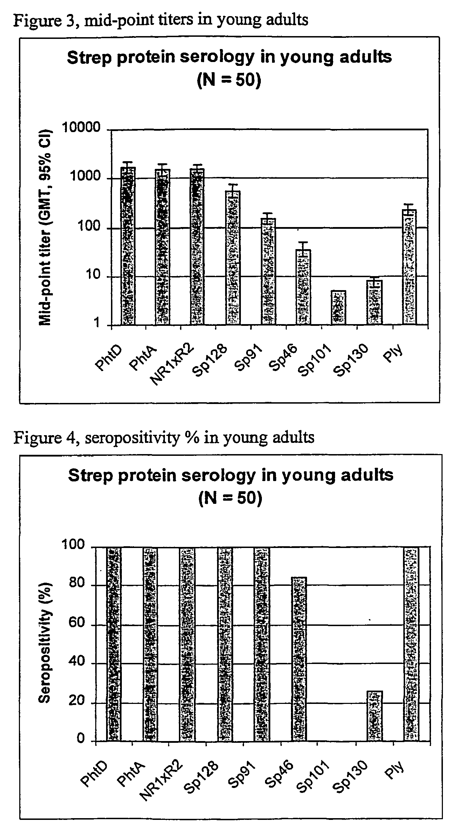

According to the geometric mean titers, PhtD, PhtA and NR1xR2 are the most immunogenic proteins in the young adult population evaluated, then followed by Sp128, Ply and Sp91. All subjects had detectable antibodies to these proteins. Lower responses were measured to Sp46, and especially to Sp130 and Sp101. PsaA was not tested (not enough serum available). (See figures 3 and 4)

### 2.2.3 Strep protein serology in elderly adults

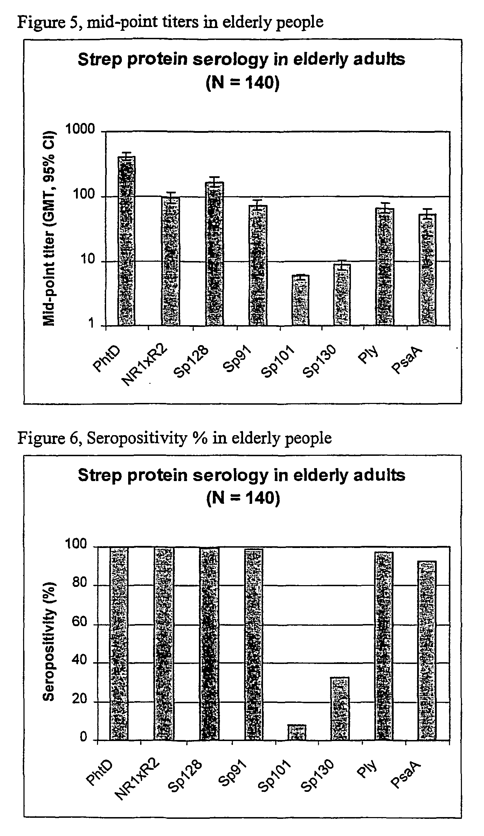

There was a clear decrease of the antibody levels to Strep proteins in elderly humans compared to young adults. In aged people, the best immunogen is PhtD, followed by Sp128, NR1xR2, Sp91, Ply and PsaA. Only marginal responses were measured to Sp101 and Sp130. Sp46 and PhtA were not tested (material availability issue). (See figures 5 and 6)

**Table 1, Geometric mean IgG concentrations (GMC), expressed as µg/ml, in elderly people**

| **Protein** | **IgG (GMC, µg/ml)** |
|---|---|
| PhtD | 19 |
| NR1xR2 | 3.5 |
| Sp91 | 2.5 |
| Ply | 2.3 |

## Claims

1. An immunogenic composition comprising at least 2 S. *pneumoniae* proteins wherein one of the proteins is PhtD and another protein is detoxified pneumolysin (Ply).

2. The immunogenic composition of claim 1 further comprising NR1xR2 or R1xR2 from CbpA or PspC.

3. The immunogenic composition of claims 1-2 wherein the pneumolysin is chemically detoxified.

4. The immunogenic composition of claims 1-2 wherein the pneumolysin is genetically detoxified.

5. The immunogenic composition of claims 1-4 additionally comprising another antigen selected from the group consisting of Hepatitis B surface antigen (HBsAg), Polio virus antigens, *Moraxella catarrhalis* outer membrane proteins, non-typeable *Haemophilus influenzae* proteins and *N. meningitidis* B outer membrane proteins.

6. The immunogenic composition of claim 5 wherein the additional antigen is a non-typeable *Haemophilus influenzae* protein.

7. The immunogenic composition of claim 6 wherein the additional antigen is Protein D from non-typeable *Haemophilus influenzae.*

8. The immunogenic composition of claims 1-7 additionally comprising an adjuvant.

9. The immunogenic composition of claim 8 wherein the adjuvant is a preferential inducer of a TH1 type response.

10. A vaccine comprising the immunogenic composition of claim 1-9.

11. Use of the vaccine of claim 10 in the manufacture of a medicament for prevention of pneumonia in patients over 55 years of age.

12. Use of the vaccine of claim 10 in the manufacture of a medicament for prevention of Otitis media in infants.

13. A method of making a vaccine as claimed in claim 10 comprising the steps of: selecting and isolating two different *S. pneumonia* proteins which are PhtD and detoxified pneumolysin (Ply); and mixing said proteins together with a pharmaceutically acceptable carrier.

## Patentansprüche

1. Immunogene Zusammensetzung, umfassend zumindest 2 *S. pneumoniae*-Proteine, wobei eines der Proteine PhtD ist und ein anderes Protein detoxifiziertes Pneumolysin (Ply) ist.

2. Immunogene Zusammensetzung gemäß Anspruch 1, ferner umfassend NR1xR2 oder R1xR2 von CbpA oder PspC.

3. Immunogene Zusammensetzung gemäß der Ansprüche 1-2, wobei das Pneumolysin chemisch detoxifiziert ist.

4. Immunogene Zusammensetzung gemäß der Ansprüche 1-2, wobei das Pneumolysin genetisch detoxifiziert ist.

5. Immunogene Zusammensetzung gemäß der Ansprüche 1-4, zusätzlich umfassend ein anderes Antigen ausgewählt aus der Gruppe bestehend aus Hepatitis-B-Oberflächenantigen (HBsAg), Polio-Virus-Antigenen, *Moraxella catarrhalis-*Außenmembranproteinen, nicht typisierbaren *Haemophilus influenzae*-Proteinen und *N. meningitidis*-B-Außenmembranproteinen.

6. Immunogene Zusammensetzung gemäß Anspruch 5, wobei das zusätzliche Antigen ein nicht typisierbares *Haemophilus influenzae-Protein* ist.

7. Immunogene Zusammensetzung gemäß Anspruch 6, wobei das zusätzliche Antigen Protein D von nicht typisierbarem *Haemophilus influenzae* ist.

8. Immunogene Zusammensetzung gemäß der Ansprüche 1-7, zusätzlich umfassend ein Adjuvanz.

9. Immunogene Zusammensetzung gemäß Anspruch 8, wobei das Adjuvanz ein bevorzugter Induktor einer TH1-Typ-Antwort ist.

10. Impfstoff, umfassend die immunogene Zusammensetzung gemäß Anspruch 1-9.

11. Verwendung des Impfstoffs gemäß Anspruch 10 zur Herstellung eines Arzneimittels für die Prävention von Pneumonie bei Patienten über 55 Jahren.

12. Verwendung des Impfstoffs gemäß Anspruch 10 zur Herstellung eines Arzneimittels für die Prävention von Otitis media bei Kindern.

13. Verfahren zum Herstellen eines Impfstoffs wie beansprucht in Anspruch 10, umfassend die Schritte:
Auswählen und Isolieren zwei verschiedener *S. pneumoniae*-Proteine, welche PhtD und detoxifiziertes Pneumolysin (Ply) sind; und Zusammenmischen der Proteine mit einem pharmazeutisch annehmbaren Träger.

## Revendications

1. Composition immunogène comprenant au moins 2 protéines de *S. pneumoniae* dans laquelle une des protéines est PhtD et l'autre est une pneumolysine détoxifiée (Ply).

2. Composition immunogène selon la revendication 1 comprenant en outre NR1xR2 ou R1xR2 issu de CbpA ou PspC.

3. Composition immunogène selon les revendications 1-2 dans laquelle la pneumolysine est chimiquement détoxifiée.

4. Composition immunogène selon les revendications 1-2 dans laquelle la pneumolysine est génétiquement détoxifiée.

5. Composition immunogène selon les revendications 1-4 comprenant en plus un autre antigène choisi dans le groupe constitué par l'antigène de surface de l'hépatite B (HbsAg), les antigènes du virus de la polio, les protéines de membrane externe de *Moraxella catarrhalis,* les protéines de souches non typables d'*Haemophilus influenzae* et les protéines de membrane externe de *N. meningitidis B.*

6. Composition immunogène selon la revendication 5 dans laquelle l'antigène supplémentaire est une protéine d'une souche non typable d'*Haemophilus influenzae.*

7. Composition immunogène selon la revendication 6 dans laquelle l'antigène supplémentaire est la protéine D issue d'une souche non typable d'*Haemophilus influenzae.*

8. Composition immunogène selon les revendications 1-7 comprenant en plus un adjuvant.

9. Composition immunogène selon la revendication 8 dans laquelle l'adjuvant est un inducteur préférentiel d'une réponse de type TH1.

10. Vaccin comprenant la composition immunogène selon les revendications 1-9.

11. Utilisation du vaccin selon la revendication 10 dans la fabrication d'un médicament destiné à prévenir la pneumonie chez les patients de plus de 55 ans.

12. Utilisation du vaccin selon la revendication 10 dans la fabrication d'un médicament destiné à prévenir l'otite moyenne chez le nourrisson.

13. Procédé de fabrication d'un vaccin selon la revendication 10 comprenant les étapes de : sélection et isolement de deux protéines différentes de *S. pneumonia* qui sont PhtD et une pneumolysine détoxifiée (Ply) ; et mélange desdites protéines avec un véhicule pharmaceutiquement acceptable.
